(19) Europäisches Patentamt
European Patent Office
Office européen des brevets

(11) **EP 0 778 020 B1**

(12) **EUROPÄISCHE PATENTSCHRIFT**

(45) Veröffentlichungstag und Bekanntmachung des Hinweises auf die Patenterteilung:
**11.09.2002 Patentblatt 2002/37**

(51) Int Cl.[7]: **A61K 7/135**

(21) Anmeldenummer: **96116145.2**

(22) Anmeldetag: **09.10.1996**

(54) **Mittel zum Bleichen von Haaren**

Hair bleaching composition

Composition pour la décoloration des cheveux

(84) Benannte Vertragsstaaten:
**DE ES FR GB IT**

(30) Priorität: **08.12.1995 DE 19545853**

(43) Veröffentlichungstag der Anmeldung:
**11.06.1997 Patentblatt 1997/24**

(73) Patentinhaber: **Wella Aktiengesellschaft**
**64274 Darmstadt (DE)**

(72) Erfinder:
• **Schmitt, Manfred**
**64646 Heppenheim (DE)**
• **Göttmann, Holger**
**64395 Brensbach-Wersau (DE)**

• **Balzer, Wolfgang R., Dr.**
**64665 Alsbach (DE)**
• **Schiemann, Hartmut, Dr.**
**36088 Hünfeld (DE)**

(56) Entgegenhaltungen:
EP-A- 0 206 128          WO-A-92/03120
DE-A- 3 814 356          GB-A- 827 331
US-A- 4 170 637

Bemerkungen:
Die Akte enthält technische Angaben, die nach dem Eingang der Anmeldung eingereicht wurden und die nicht in dieser Patentschrift enthalten sind.

**Beschreibung**

Gegenstand der vorliegenden Erfindung ist ein aus zwei Komponenten erhältliches Mittel zum Entfärben oder Blondieren von Haaren, insbesondere menschlichen Haaren.

Zum Entfärben oder Blondieren von Haaren werden üblicherweise oxidierende Zubereitungen verwendet, welche durch Auflösen eines sogenannten Blondierpulvers (Pulvergemisch aus Alkalisalzen und anorganischen Persalzen, wie zum Beispiel Natrium- oder Ammoniumpersulfat) in einer wäßrigen Wasserstoffperoxidlösung erhalten werden.

Die Verwendung derartiger Blondierpulver, welche notwendigerweise aus mehreren Bestandteilen zusammengesetzt sind, bringt jedoch eine Vielzahl von Nachteilen mit sich. So kommt es durch die Verwendung von Rohstoffen mit unterschiedlicher Dichte häufig während des Transportes oder der Lagerung zur Trennung der unterschiedlichen Pulverbestandteile, wobei sich die schweren Pulverbestandteile im unteren Teil und die leichteren Pulverbestandteile im oberen Teil des Behältnisses ansammeln. Diese Entmischung hat zur Folge, daß die gleiche Pulvermenge je nach ihrem Entnahmeort eine unterschiedliche chemische Zusammensetzung und somit auch eine unterschiedliche Blondierwirkung aufweisen kann.

Um dieser Entmischung entgegenzuwirken, ist es erforderlich, vor jeder Pulverentnahme das Pulver gründlich zu schütteln, was der Verwender in der Regel jedoch nicht tut.

Eine Entmischung kann auch durch den Einsatz von Pulvergemischen mit sehr kleiner Korngröße verhindert werden. Dies hat jedoch den Nachteil, daß solche Pulvergemische - insbesondere beim Öffnen der Behältnisse, bei der Entnahme des Pulvers oder beim Vermischen mit der Wasserstoffperoxidlösung - zu einer starken Staubentwicklung neigen, was zu einer Reizung der Atmungsorgane führen kann. Weiterhin besitzen derartige Pulvergemische aufgrund der geringen Korngröße eine große Oberfläche, wodurch eine Aufnahme von Feuchigkeit beim Öffnen und Schließen des Behältnisses und damit eine Verringerung der Blondierwirkung infolge der Desaktivierung des Sauerstoffträgers begünstigt wird.

Die Zubereitung der gebrauchsfertigen Mischung erfolgt zum einen durch Verrühren der Bestandteile in einer Schale, zum anderen durch Vermischen in einer Anschüttelflasche, wobei insbesondere das Anschütteln oftmals mit einer lästigen Staubentwicklung beim Einfüllen der Komponenten in die Schüttelflasche verbunden ist.

Es wurden bereits zahlreiche Versuche unternommen, dieses Problem zu lösen.

So wird in der DE-OS 40 26 235 vorgeschlagen, anstelle eines Blondierpulvers eine Mischung, bestehend aus einem Granulat der anorganischen Persulfate und einem Granulat der übrigen Bestandteile des Blondiermittels, zu verwenden. Hierdurch kann zwar das Problem der Staubentwicklung behoben werden, das Problem der Entmischung kann auf diesem Wege jedoch nicht gelöst werden, da es technisch äußerst schwierig ist, Einzelgranulate mit identischer und konstanter Korngröße oder Schüttgewicht herzustellen. Weiterhin kann es aufgrund der unterschiedlichen Löslichkeit der einzelnen Granulate zu einer Beeinträchtigung der Blondierwirkung kommen. Es erscheint zudem auch aus ökonomischen Gesichtspunkten wenig sinnvoll, anstelle eines einzelnen Granulates eine Mischung aus mehreren Granulaten herzustellen.

In der DE-AS 20 23 922 wird empfohlen, ein Granulat anstelle des Pulvers zu verwenden. Dieses Granulat soll durch Besprühen des alle erforderlichen Bestandteile enthaltenden Blondierpulvers mit einer wäßrigen, alkoholischen oder wäßrig-alkoholischen Polymer-Lösung in einem geeigneten Mischer hergestellt werden.

Bei diesem Granulierverfahren kommt es jedoch zu einem starken Ammoniakverlust, wodurch die Blondierwirkung des Granulats verschlechtert wird. Dieser Ammoniakverlust soll durch die Erhöhung des Ammoniumsalzanteils in dem eingesetzten Pulver bzw. durch den Zusatz von Ammoniak zu der Polymerlösung kompensiert werden. Da der verfahrensbedingte Ammoniakverlust bei diesem Granulationsverfahren stark schwankt, ist es mit diesem Verfahren nicht möglich, ein Granulat mit einer konstanten chemischen Zusammensetzung herzustellen.

Weiterhin kann auch bei diesen Mitteln die Staubfreiheit nicht völlig gewährleistet werden, da durch Reibung der Granulatteilchen aneinander, z. B. während des Transportes, Feinstaub gebildet wird.

Eine Verwendung solcher Blondiergranulate in einer Anschüttelflasche ist wegen der schlechten Löslichkeit der Granulate nicht möglich.

In der EP-PS 0 560 088 wird ein pulverförmiges Mittel zum Blondieren von Haaren beschrieben, bei dem zur Verhinderung der Staubbildung ein Öl oder flüssiges Wachs zugesetzt wird. Aber auch hierdurch kann eine völlige Staubfreiheit nicht erreicht werden. Zudem ergibt sich durch den Wassergehalt der eingesetzten pulverförmigen Rohstoffe und die durch den Ölzusatz bedingte, kompakte Pulverform eine Desaktivierung der Sauerstoffträger, wodurch das Produkt instabil wird und seine Bleichwirkung verliert.

Weiterhin sind derartige Blondiermittel aufgrund ihres spezifischen Gewichtes und ihres hydrophoben Charakters für die Verwendung in der Auftrageflasche ungeeignet, da das Pulver in der Wasserstoffperoxidlösung nach unten sinkt und nicht ausreichend benetzt wird, wodurch eine inhomogene Mischung mit einem hohen Anteil an ungelöstem Pulver erhalten wird, durch die die Austrittsdüse der Auftrageflasche verstopft wird. Der Zusatz von Tensiden zur Verbesserung der Löslichkeit des Pulvers ist ebenfalls problematisch, da hierdurch die Lagerfähigkeit des Pulvers beeinträchtigt wird.

Aus der DE-OS 38 14 356 sind breiartige Blondiermittel bekannt, welche anorganische oder organische Verdicker, beispielsweise Parrafin- oder Montanwachse, disperse Kieselsäure, speziell behandelte Tone und Silikate oder hydrierte Rizinusölderivate, enthalten. Derartige Zubereitungen ermögliche zwar eine weitgehende Staubfreiheit, jedoch werden auch durch diese Mittel nicht alle an Blondiermittelmittel gestellten Anforderungen in jeder Hinsicht erfüllt.

Es bestand daher die Aufgabe, ein lagerstabiles pastenförmiges Mittel zum Entfärben oder Blondieren von Humanhaaren zur Verfügung zu stellen, welches vor Gebrauch durch einfaches Schütteln oder Anrühren mit einer flüssigen Wasserstoffperoxidlösung oder einer wasserstoffperoxidhaltigen Öl-in-Wasser-Emulsion vermischt wird und neben seiner absolut staubfreien Darreichungs- und Anwendungsform stärkste Blondierleistung bei gleichzeitig hervorragender Lagerstabilität gewährleistet.

Überraschend wurde nunmehr gefunden, daß die vorstehend beschriebenen Nachteile vermieden werden können, wenn man ein Blondiermittel in Form einer auf einer speziellen Kombination von Inhaltsstoffen basierenden Suspension oder Dispersion verwendet.

Gegenstand der vorliegenden Erfindung ist daher ein Mittel zum Entfärben oder Blondieren von Haaren, welches unmittelbar vor der Anwendung durch Vermischen einer cremeförmigen Blondiermittelsuspension mit einem Oxidationsmittel hergestellt wird und dadurch gekennzeichnet ist, daß die Blondiermittelsuspension eine Mischung aus (a) mindestens einem anorganischen Persalz, (b) mindestens einem alkalisch reagierenden Salz, (c) einer Verdickerkombination, bestehend aus einem Acrylsäurepolymer und mindestens einem Polymer aus der Gruppe der Cellulosen, Alginate und Polysaccharide, (d) mindestens einem Öl oder Wachs sowie (e) gegebenenfalls Hilfs- und Zusatzstoffen darstellt.

Als anorganisches Persalz werden vorzugsweise Persulfate, beispielsweise Natriumpersulfat, Kaliumpersulfat, Ammoniumpersulfat oder Mischungen dieser Persulfate, eingesetzt, wobei die Persulfate in der Blondiermittelsuspension in einer Gesamtmenge von vorzugsweise 30 bis 65 Gewichtsprozent, insbesondere 35 bis 55 Gewichtsprozent, enthalten sind.

Als alkalisch reagierendes Salz werden vorzugsweise in wäßriger Lösung alkalisch reagierende Alkali- oder Erdalkalisalze, beispielsweise Natriumcarbonat, Natriumhydrogencarbonat, Magnesiumcarbonat, Ammoniumcarbonat, Ammoniumhydrogencarbonat, Natriumsilikate oder Mischungen dieser Salze, verwendet, wobei diese Salze in der Blondiermittelsuspension in einer Gesamtmenge von vorzugsweise 15 bis 45 Gewichtsprozent, insbesondere 18 bis 35 Gewichtsprozent, enthalten sind.

Als Polymer aus der Gruppe der Cellulosen, Alginate und Polysaccharide werden vorzugsweise Methylcellulosen, Ethylcellulosen, Hydroxyethylcellulosen, Methylhydroxyethylcellulosen, Methylhydroxypropylcellulosen, Carboxymethylcellulosen, Alginsäure, Natriumalginat, Ammoniumalginat, Calciumalginat, Gummi Arabicum, Guar Gum oder Xanthan Gum, alleine oder in Kombination, verwendet, wobei die Verwendung von anquellverzögernden Methylhydroxyethylcellulosen oder einer Kombination von Natriumalginaten mit Polysacchariden oder Cellulosen besonders bevorzugt ist.

Als besonders vorteilhaft hat sich hierbei die Kombination von Natriumalginaten mit Xanthan Gum sowie die Kombination von Natriumalginaten mit Methylhydroxyethylcellulose in einem Verhältnis von 1 zu 3 bis 3 zu 1, insbesondere von 1 zu 2 bis 2 zu 1, erwiesen.

Bezogen auf die Gesamtmenge der Blondiermittelsuspension werden die Cellulosen in einer Menge von 0,1 bis 20 Gewichtsprozent, vorzugsweise 0,2 bis 15 Gewichtsprozent, eingesetzt, wobei eine Einsatzmenge von 0,5 bis 12 Gewichtsprozent besonders bevorzugt ist.

Die Einsatzmenge für die Alginate und Polysaccharide beträgt, bezogen auf die Gesamtmenge der Blondiermittelsuspension, jeweils 0,1 bis 15 Gewichtsprozent, vorzugsweise 0,2 bis 12 Gewichtsprozent und insbesondere 0,5 bis 10 Gewichtsprozent.

Als Acrylsäurepolymer werden vorzugsweise hochmolekulare Acrylsäurepolymere mit einem Molekulargewicht von etwa 1.250.000 bis 4.000.000, beispielsweise die Handelsprodukte Carbopol® 940, Carbopol® 941, Carbopol® 954 und Carbopol® 981 der Firma BF Goodrich/USA oder die Handesprodukte Acrisint® 410, Synthalen® L und Synthalen® K der Firma 3V-Sigma/USA, eingesetzt.

Das Acrylsäurepolymer wird, bezogen auf die Gesamtmenge der Blondiermittelsuspension, vorzugsweise in einer Menge von 0,1 bis 3 Gewichtsprozent, insbesondere 0,1 bis 2 Gewichtsprozent, eingesetzt.

Die Verdickerkombination (c) ist in der Blondiermittelsuspension in einer Gesamtmenge von etwa 0,5 bis 20 Gewichtsprozent, vorzugsweise 1,5 bis 17 Gewichtsprozent, enthalten.

Als Öl oder Wachs eignen sich insbesondere flüssige oder wachsförmige, langkettige, hydrophobe Fettsäureester. Als flüssige, langkettige, hydrophobe Fettsäureester können beispielsweise Isopropylpalmitat, Octylpalmitat und Isocetylpalmitat verwendet werden. Als wachsförmiger, langkettiger, hydrophober Fettsäureester eignet sich insbesondere Bienenwachs. Besonders bevorzugt ist hierbei die Verwendung einer Kombination aus Bienenwachs und Fettsäureestern, insbesondere Isopropylpalmitat.

Das Bienenwachs wird, bezogen auf die Gesamtmenge der Blondiermittelsuspension, vorzugsweise in einer Menge von 0,5 bis 10 Gewichtsprozent, insbesondere 1 bis 8 Gewichtsprozent, eingesetzt, wobei die Gesamtmenge der in der Blondiermittelsuspension enthaltenen organischen Öle und Wachse 26,5 bis 35 Gewichtsprozent, vorzugsweise 26,5 bis 29 Gewichtsprozent, beträgt.

Zusätzlich kann die cremeförmige Blondiermittelsuspension weitere für derartige Zubereitungen übliche Zusatzstoffe, wie zum Beispiel Siliciumdioxid, Titandioxid, Chelatisierungsmittel für Schwermetallionen, beispielsweise Ethylendiaminotetraessigsäure, Anfärbestoffe, beispielsweise Ultramarinfarbstoffe oder saure Farbstoffe, oder Parfüme, enthalten, wobei diese Zusatzstoffe in den für solche Mittel üblichen Einsatzmengen, beispielsweise das Siliciumdioxid und das Chelatisierungsmittel jeweils in einer Menge von 0,1 bis 3 Gewichtsprozent, und die Anfärbestoffe und Parfüme jeweils in einer Menge von 0,01 bis 1 Gewichtsprozent, eingesetzt werden.

Vorzugsweise enthält das erfindungsgemäße Blondiermittel keine Tenside und ist wasserfrei. Ein Wassergehalt von maximal bis zu 2,5 Gewichtsprozent ist jedoch zulässig.

Vor der Anwendung wird die cremeförmige Blondiermittelsuspension mit einem Oxidationsmittel, vorzugsweise einer wäßrigen Wasserstoffperoxidlösung oder wasserstoffperoxidhaltigen Öl-in-Wasser-Emulsion, zu einem auftragefähigen Blondierbrei vermischt, wobei dieses Vermischen in einer Schale oder durch Anschütteln in einer Auftrageflasche erfolgen kann.

Das Mischungsverhältnis von Blondiermittelsuspension zu Oxidationsmittel beträgt bei Verwendung einer 6- bis 12-prozentigen Wasserstoffperoxid-Lösung oder -Emulsion 1 zu 1 bis 1 zu 3.

Das so erhaltene gebrauchsfertige Mittel zum Entfärben oder Blondieren von Haaren wird auf das Haar gleichmäßig aufgetragen und nach einer Einwirkungszeit von 15 bis 60 Minuten bei Raumtemperatur (20-25°C) beziehungsweise von 10 bis 50 Minuten bei Wäremeinwirkung (30-50°C) mit Wasser ausgespült.

Die cremeförmige Blondiermittelsuspension kann je nach ihrer Viskosität in Tuben oder Tiegel abgefüllt werden und ist äußerst leicht mit dem Oxidationsmittel vermischbar, was aus der leichten Anschüttelbarkeit und der kurzen Anmischzeit von unter 20 Sekunden für 100 g der gebrauchsfertigen Zubereitung ersichtlich ist. Neben der anwendungsfreundlichen Produktviskosität und der leichten Anmischbarkeit zeichnet sich das erfindungsgemäße Mittel durch eine hervorragende Auftragefähigkeit, Verteilbarkeit und Haftung am Haar sowie eine sehr hohe Blondierwirkung und ein breites Anwendungsspektrum aus.

Die nachfolgenden Beispiele sollen den Gegenstand näher erläutern, ohne diesen hierauf zu beschränken.

**Beispiele**

**Beispiel 1:** Cremeförmige Blondiermittelsuspension

| | |
|---|---|
| 25,0 g | Kaliumpersulfat |
| 18,0 g | Ammoniumpersulfat |
| 23,0 g | Natriummetasilikat |
| 2,0 g | Natriumalginat |
| 2,0 g | Xanthan Gum |
| 0,5 g | Acrylsäurepolymer (CTFA-Carbomer) |
| 26,5 g | Isopropylpalmitat |
| 2,5 g | Bienenwachs |
| 0,5 g | Ethylendiaminotetraessigsäure |
| 100,0 g | |

**Herstellung der Blondiermittelsuspension im Technikummaßstab (10 kg Ansatz)**

Das Isopropylpalmitat und das Bienenwachs werden in einem Unimix-Rührwerk (Type SR 15) vorgelegt und bei etwa 54° C geschmolzen und sodann unter Rühren auf etwa 30° C abgekühlt. Die übrigen Bestandteile werden zunächst in einem Lödige-Mischer (Type FM 50 E 17) 6 Minuten lang mit Mixer und Chopper homogen miteinander vermischt und sodann zu dem Gemisch aus Isopropylpalmitat und Bienenwachs gegeben. Nach einer Rührzeit von 15 Minuten wird eine homogene cremeförmige Blondiermittelsuspension erhalten, die beispielsweise in handelsübliche, mit einem Innenlack versehene Aluminiumtuben abgefüllt werden kann.

**Anwendungsbeispiel a)**

25 g der Blondiermittelsuspension werden mit 25 g einer 9-prozentigen wasserstoffperoxidhaltigen Öl-in-

# EP 0 778 020 B1

Wasser-Emulsion der folgenden Zusammensetzung

| | |
|---|---|
| 18,0 | Wasserstoffperoxid (50-prozentige wäßrige Lösung) |
| 2,0 | Cetylstearylalkohol |
| 0,2 | Lanolinalkohol |
| 0,1 | Phosphorsäure (85-prozentig) |
| 79,7 | Wasser |
| 100,00 g | |

in einer Schale mit einem Pinsel homogen verrührt.

Das erhaltene, breiartige Blondiermittel wird auf mittelbraunes Haar gleichmäßig aufgetragen und nach einer Einwirkungszeit von 30 Minuten bei Raumtemperatur mit warmem Wasser abgespült und getrocknet. Das so behandelte Haar ist auf einen hellblonden Farbton aufgehellt worden.

**Anwendungsbeispiel b)**

25 g der vorstehend beschriebenen Blondiermittelsuspension werden mit 37,5 g einer 6-prozentigen wäßrigen Wasserstoffperoxidlösung in einer Schale mit einem Pinsel homogen verrührt. Es ist jedoch auch möglich, die Wasserstoffperoxidlösung in einer Auftrageflasche vorzulegen und mit der Blondiermittelsuspension zum gebrauchsfertigen Blondiermittel anzuschütteln.

Das Blondiermittel wird gleichmäßig auf das aufzuhellende Haar aufgetragen und nach einer Einwirkungszeit von 40 Minuten bei Raumtemperatur mit Wasser abgespült. Sodann wird das Haar getrocknet. Der Aufhellungsgrad beträgt etwa 4 Tonstufen.

**Anwendungsbeispiel c)**

25 g der vorstehend beschriebenen Blondiermittelsuspension werden mit 75 g einer 6-prozentigen Wasserstoffperoxidemulsion in einer Auftrageflasche 10 bis 15 Sekunden geschüttelt.

Anschließend wird das Blondiermittel mittels der Auftrageflasche gleichmäßig auf das aufzuhellende Haar aufgetragen.

Nach einer Einwirkungszeit von 30 Minuten bei Raumtemperatur (20 bis 30° C) wird das Haar mit warmem Wasser gründlich ausgespült und getrocknet.

Der Aufhellungsgrad beträgt 3 Tonstufen und kann bei Verlängerung der Einwirkungszeit um 20 Minuten um 1 bis 2 Tonstufen erhöht werden.

Alle Prozentangaben stellen, soweit nicht anders angegeben, Gewichtsprozente dar.

**Patentansprüche**

1. Mittel zum Entfärben oder Blondieren von Haaren, das unmittelbar vor der Anwendung durch Vermischen einer cremeförmigen Blondiermittelsuspension mit einem Oxidationsmittel hergestellt wird, **dadurch gekennzeichnet, daß** die Blondiermittelsuspension eine Mischung aus (a) mindestens einem anorganischen Persalz, (b) mindestens einem alkalisch reagierenden Salz, (c) einer Verdickerkombination, bestehend aus einem Acrylsäurepolymer und mindestens einem Polymer aus der Gruppe der Cellulosen, Alginate und Polysaccharide, (d) mindestens einem Öl oder Wachs und (e) gegebenenfalls Hilfs- und Zusatzstoffen darstellt.

2. Mittel nach Anspruch 1, **dadurch gekennzeichnet, daß** das Persalz ausgewählt ist aus Natriumpersulfat, Kaliumpersulfat, Ammoniumpersulfat und Mischungen dieser Persulfate.

3. Mittel nach Anspruch 1 oder 2, **dadurch gekennzeichnet, daß** das Persalz in der Blondiermittelsuspension in einer Gesamtmenge von 30 bis 65 Gewichtsprozent enthalten ist.

4. Mittel nach einem der Ansprüche 1 bis 3, **dadurch gekennzeichnet, daß** das alkalisch reagierende Salz ausgewählt ist aus Natriumcarbonat, Natriumhydrogencarbonat, Magnesiumcarbonat, Ammoniumcarbonat, Ammoniumhydrogencarbonat, Natriumsilikaten und Mischungen dieser Salze ausgewählt ist.

5. Mittel nach einem der Ansprüche 1 bis 4, **dadurch gekennzeichnet, daß** das alkalisch reagierende Salz in der Blondiermittelsuspension in einer Gesamtmenge von 15 bis 45 Gewichtsprozent enthalten ist.

6.  Mittel nach einem der Ansprüche 1 bis 5, **dadurch gekennzeichnet, daß** als Cellulosen, Alginate oder Polysaccharide Methylcellulosen, Ethylcellulosen, Hydroxyethylcellulosen, Methylhydroxyethylcellulosen, Methylhydroxypropylcellulosen, Carboxymethylcellulosen, Alginsäure, Natriumalginat, Ammoniumalginat, Calciumalginat, Gummi Arabicum, Guar Gum oder Xanthan Gum verwendet werden.

7.  Mittel nach einem der Ansprüche 1 bis 6, **dadurch gekennzeichnet, daß** die Verdickerkombination in der Blondiermittelsuspension in einer Gesamtmenge von 0,5 bis 20 Gewichtsprozent enthalten ist.

8.  Mittel nach einem der Ansprüche 1 bis 7, **dadurch gekennzeichnet, daß** das Öl oder Wachs ausgewählt ist aus Isopropylpalmitat, Octylpalmitat, Isocetylpalmitat, Bienenwachs und Mischungen dieser Verbindungen.

9.  Mittel nach einem der Ansprüche 1 bis 8, **dadurch gekennzeichnet, daß** das Öl oder Wachs in der Blondiermittelsuspension in einer Gesamtmenge von 26,5 bis 35 Gewichtsprozent enthalten ist.

10. Mittel nach einem der Ansprüche 1 bis 9, **dadurch gekennzeichnet, daß** als Oxidationsmittel eine 6-bis 12-prozentige wäßrige Wasserstoffperoxidlösung oder Wasserstoffperoxidemulsion verwendet wird.

**Claims**

1.  Agent for bleaching hair or dyeing it blonde, which may be produced immediately before application by mixing a blonding agent suspension in creme form with an oxidising agent, **characterised in that** the blonding agent is a mixture of (a) at least one inorganic persalt, (b) at least one salt which reacts in an alkaline manner, (c) a thickener combination, consisting of an acrylic acid polymer and at least one polymer from the group of celluloses, alginates and polysaccharides, (d) at least one oil or wax and (e), optionally, auxiliary substances and additives.

2.  Agent according to Claim 1, **characterised in that** the persalt is selected from sodium persulphate, potassium persulphate, ammonium persulphate and mixtures of these persulphates.

3.  Agent according to Claim 1 or 2, **characterised in that** the persalt is contained in . the blonding agent suspension in a total amount of from 30 to 65 weight per cent.

4.  Agent according to any one of Claims 1 to 3, **characterised in that** the salt, which reacts in an alkaline manner is selected from sodium carbonate, sodium hydrogen carbonate, magnesium carbonate, ammonium carbonate, ammonium hydrogen carbonate, sodium silicates and mixtures of these salts.

5.  Agent according to any one of Claims 1 to 4, **characterised in that** the salt which reacts in an alkaline manner is contained in the blonding agent suspension in a total amount of from 15 to 45 weight per cent.

6.  Agent according to any one of Claims 1 to 5, **characterised in that** methylcelluloses, ethylcelluloses, hydroxyethylcelluloses, methylhydroxyethylcelluloses, methylhydroxypropylcelluloses, carboxymethylcelluloses, alginates, sodium alginate, ammonium alginate, calcium alginate, gum arabic, guar gum or xanthan gum are used as celluloses, alginates or polysaccharides.

7.  Agent according to any one of Claims 1 to 6, **characterised in that** the thickener combination is contained in the blonding agent suspension in a total amount of from 0.5 to 20 weight per cent.

8.  Agent according to any one of Claims 1 to 7, **characterised in that** the oil or wax is selected from isopropylpalmitate, octylpalmitate, isocetylpalmitate, beeswax and mixtures of these compounds.

9.  Agent according to one of Claims 1 to 8, **characterised in that** the oil or wax in the blonding agent suspension is contained in a total amount of 26.5 to 35 weight per cent.

10. Agent according to one of Claims 1 to 9, **characterised in that** a 6 to 12 per cent aqueous hydrogen peroxide solution or hydrogen peroxide emulsion is used as the oxidation agent.

**Revendications**

1. Agent de blanchiment ou de décoloration des cheveux, préparé directement avant application par mélange d'une suspension d'agent de décoloration se présentant sous forme de crème, avec un agent d'oxydation, **caractérisé en ce que** la suspension d'agent de décoloration contient un mélange constitué de (a) au moins un persel inorganique, (b) au moins un sel réagissant de façon alcaline, (c) une combinaison épaississante, constituée d'un polymère d'acide acrylique et d'au moins un polymère issu du groupe des celluloses, alginates ou polysaccharides, (d) au moins une huile ou une cire et (e) le cas échéant des substances auxiliaires et additionnelles.

2. Agent selon la revendication 1, **caractérisé en ce que** le persel est sélectionné parmi le persulfate de sodium, persulfate de potassium, persulfate d'ammonium et des mélanges de ces persulfates.

3. Agent selon la revendication 1 ou 2, **caractérisé en ce que** le persel est contenu dans la suspension d'agent de décoloration en une quantité totale comprise dans la plage allant 30 à 65 pour-cent en poids.

4. Agent selon l'une des revendications 1 à 3, **caractérisé en ce que** le sel réagissant de façon alcaline est sélectionné dans le groupe constitué de carbonate de sodium, carbonate hydrogéné de sodium, carbonate de magnésium, carbonate d'ammonium, carbonate hydrogéné d'ammonium, silicates de sodium et des mélanges de ces sels.

5. Agent selon l'une des revendications 1 à 4, **caractérisé en ce que** le sel réagissant de façon alcaline est contenu dans la suspension d'agent de décoloration en une quantité totale comprise dans la plage allant de 15 à 45 pour-cent en poids.

6. Agent selon l'une des revendications 1 à 5, **caractérisé en ce qu'**on utilise comme celluloses, alginates ou polysaccharides des méthylcelluloses, éthylcelluloses, hydroxyéthylcelluloses, méthylhydroxyéthylcelluloses, méthylhydroxypropylcelluloses, carboxyméthylcelluloses, de l'acide alginique, de l'alginate de sodium, alginate d'ammonium, alginate de calcium, de la gomme arabique, de la gomme de Guar ou de la gomme de Xanthan.

7. Agent selon l'une des revendications 1 à 6, **caractérisé en ce que** la combinaison épaississante est contenue dans la suspension d'agent de décoloration en une quantité totale comprise dans la plage allant de 0,5 à 20 pour-cent en poids.

8. Agent selon l'une des revendications 1 à 7, **caractérisé en ce que** l'huile ou la cire est sélectionnée parmi 1' isopropylpalmitate, l'octylpalmitate, l'isocétylpalmitate, la cire d'abeilles et des mélanges de ces combinaisons.

9. Agent selon l'une des revendications 1 à 8, **caractérisé en ce que** l'huile ou la cire est contenue dans l'agent de décoloration une quantité totale comprise dans la plage allant de 26,5 à 35 pour-cent en poids.

10. Agent selon l'une des revendications 1 à 9, **caractérisé en ce que** l'on utilise comme agent d'oxydation une solution aqueuse de peroxyde d'hydrogène ou une émulsion aqueuse de peroxyde d'hydrogène de titre 6 à 12 pour-cent